(19) Europäisches Patentamt
European Patent Office
Office européen des brevets

(11) Publication number: **0 357 956**
**A2**

(12) **EUROPEAN PATENT APPLICATION**

(21) Application number: **89114293.7**

(22) Date of filing: **17.07.84**

(51) Int. Cl.5: **C07D 295/02 , C07D 295/14 , C07D 295/10 , C07D 295/08 , C07D 295/18 , C07D 213/38 , C07D 307/14 , C07D 333/20 , A61K 31/495 , A61K 31/33**

(30) Priority: **02.08.83 ES 524680**

(43) Date of publication of application:
**14.03.90 Bulletin 90/11**

(60) Publication number of the earlier application in accordance with Art.76 EPC: **0 132 764**

(84) Designated Contracting States:
**AT BE CH DE FR GB IT LI NL SE**

(71) Applicant: **SOCIEDAD ESPANOLA DE ESPECIALIDADES FARMACO-TERAPEUTICAS, S.A.**
**173, Avda. San Antonio Ma Claret**
**Barcelona-26(ES)**

(72) Inventor: **Cirera, Xavier D.**
**271, Avenida Rep. Argentina**
**Barcelona-23(ES)**
Inventor: **Andreoli, Romeo R.**
**6, Calle Roca i Batlle**
**Barcelona-23(ES)**
Inventor: **Lloveras, Pedro P.**
**94, Calle Viveret**
**Tarrasa (Barcelona)(ES)**
Inventor: **Bruseghini, Leónida**
**5, Calle Caponata**
**Barcelona-34(ES)**
Inventor: **Irurre, José P.**
**184, Calle Mayor de Sarriá**
**Barcelona-17(ES)**

(74) Representative: **Dipl.-Ing. Schwabe, Dr. Dr. Sandmair, Dr. Marx**
**Stuntzstrasse 16**
**D-8000 München 80(DE)**

(54) **Vasodilatory dihydrobenzocycloheptiliden-ethyl-piperazine derivatives and process for preparation thereof.**

(57) It is disclosed that certain dihydrobenzocycloheptiliden-ethyl-piperazine derivatives having the structural formula

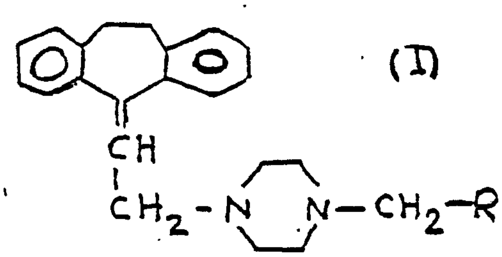

(I)

wherein the group R is preferably selected from ethylene, phenyl and 2-thienyl possess a valuable vasodilatory activity.

EP 0 357 956 A2

## Vasodilatory dihydrobenzocycloheptiliden-ethyl-piperazine derivatives and process for preparation thereof.

The present invention concerns new compounds derived from dihydrobenzocycloheptiliden-ethyl-piperazine having vasodilatory activity, process for preparing these compounds and to pharmaceutical compositions containing these compounds.

In J. Org. Chem. 27, 230-240 (1962) an antidepressive activity of dibenzo-[a,d] -1,4-cycloheptadiene derivatives is disclosed.

US-A-4381305 discloses a vascular antispasmodic and antiallergic activity of certain 1,2 ethylenediamine derivatives.

US-A-4308387 discloses certain diphenylbutyl-piperazine-carboxamide derivatives which possess antipsychotic properties.

CH-A-383997 discloses a method for preparing certain new ethylidene derivatives of fluorene or thioxanthene having antihistaminic activity.

EP-A-0 056 616 discloses certain cycloheptene derivatives having antihistaminic activity.

The dihydrobenzocycloheptiliden-ethylpiperazine derivatives according to the present invention possess the following structural formula

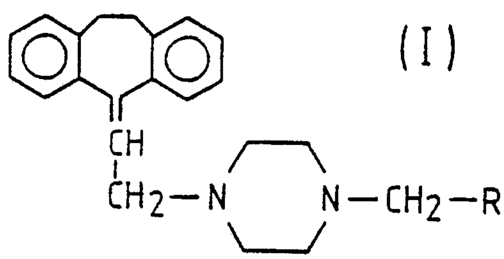

(I)

in which R represents:

$-CH=CH_2$ ; $-CH_2OH$ ; $-CO_2H$ ; $-CO_2C_2H_5$ ; $-CH=CH-CO_2CH_3$ ;

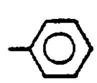 ; 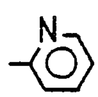 ; 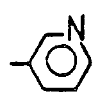 ; 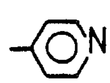 ; 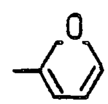 ; 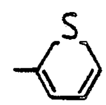 ;

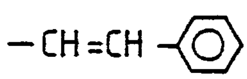 ; 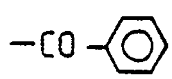 ; 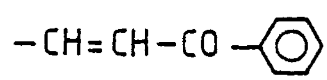 ;

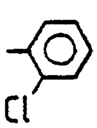 ; 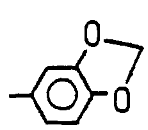 ; 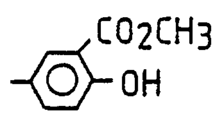 ; 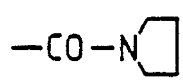 ;

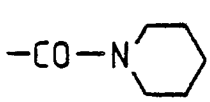 ; 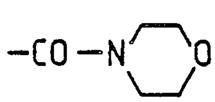 ; 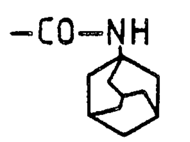 ; 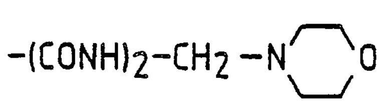

The invention also refers to the salts and other derivatives of pharmacological interest of the compound (I) including N-oxydes and quaternary ammonium salts, to the process for the obtention of said compounds

and their derivatives, and to their therapeutic applications.

Said compounds possess a marked vasodilatory activity.

PROCESS OF OBTENTION OF THE NEW COMPOUNDS OF GENERAL FORMULA I, AND OF THEIR DERIVATIVES.

The process for the obtention of the compounds defined by the general formula(I) is characterized by the reaction of piperazine, of general formula (II)

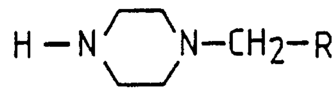

(II)

where R possess the same significance as in the general formula I, with a halogen derivative which has the formula III

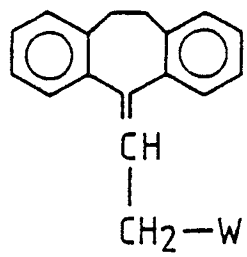

(III)

W: Cl, Br

The reaction is carried out in an inert solvent in presence of a hydrogen halide binder, which may be in inorganic or organic base, or an excess of the original amine.

An alternative form of isolation of the compounds (I) may be by formation of salts with mineral acids such as hydrochloric, sulphuric or nitric acid, or with organic acids such as, for example, oxalic, salycylic, citric, maleic or fumaric acid. The utilization of hydrochloric or maleic acid is preferable, due to their pharmacological properties.

The compounds of formula (I) object of this invention are designed briefly by the references as follows: the alphabetic expression "WAS-..." followed by a specific number which is exclusive for each formula.

EXAMPLES OF CHEMICAL SYNTHESIS

Example 1.-Obtention of N-benzyl-N′-2-(10,11-dihydrodibenzo-(a,d)-cyclohept-5-yliden)-ethylpiperazine.

This compound is briefly designated as WAS-4206.

A mixture of 5.10 gr (20 mmol) of 2-chloro-1-(10,11-dihydrobenzo-(a,d)-cyclohept-5-yliden)-ethane and 3.52 gr (20 mmol) of N-benzylpiperazine was refluxed in 100 ml acetonitril for 4 hr. in presence of 2.52 gr (3 mmol) of sodium bicarbonate.

The mixture was cooled, filtered, and the solvent was eliminated, crystallizing the residue from 10 ml of acetone. 3.78 gr of N-benzyl-N′-2-(10,11-dihydro-dibenzo-(a,d)-cyclohept-5-yliden)-ethylpiperazine were obtained. Yield: 48 %. Analytical data: Melting point: 128-130ºC IR : 3055, 3015, 2930, 2800, 1600, 1485, 1450, 1145 1010, 770, 755, 740 y 700. NMR: 7,25/sc(13H) ; 6,0/t(1H); 3,50/s(2H) ; 3,10/sc(8H) ; 2,50/s(4H) ; 2,35/sc(2H).

Example 2.-Obtention of N-2-(10,11-dihydrobenzo-(a,d)-cyclohept-5-yliden)-ethyl-N′-(2-thenyl)-piperazine.

This compound is briefly designated as WAS-4226.

A mixture of 5.10 gr (20 mmol) of 2-chloro-1-(10,11-dihydrodibenzo-(a,d)-cyclohept-5-yliden)-ethane and 3.64 gr (20 mmol) of N-2-thenylpiperazine was refluxed in 100 ml of chloroform for 6 hr. in presence of 2.52 gr (30 mmol) of sodium bicarbonate.

The mixture was cooled, filtered, and the solvent was eliminated, and the residue suspended in 50 ml of acetone and treated with an excess of saturated maleic acid in acetone.

The precipitate was filtered and recrylstallized from water.

6.24 gr of N-2-(10,11-dihydrodibenzo-(a,d)-cyclohept-5-yliden)-ethyl-N′-2-(thenyl)-piperazine associated at 1:2 with maleic acid were obtained. Yield: 50 %. Analytical data: Melting point: 202ºC (dec.) IR : 3070, 3010, 1690, 1625, 870, 775, 760, 745, 650. NMR: 8,8/sa(4H) ; 7,6/sc(1H) ; 7,3/sc(10H) ; 6,25/s(1H) ; 6,05/t-(1H); 2,5-4,0/sc(16H).

In the TABLE 1 which follows, examples 3 to 22 for obtention of the compounds object of the present invention are set out. In each case, the specific chemical structure and the analytical data which correspond to each of said compounds are known, and the method of synthesis followed are specified with reference to the detailled aforementioned examples 1 to 2.

In said Examples 1 to 22 the abreviature IR refers to the infrared spectrum and the abreviature NMR signifies the proton nuclear magnetic resonance spectrum. In said NMR analyses the following are shown:

In the first place, the position of the signal in parts per million (scale δ).

Afterwards, the form or multiplicity of the signal is indicated by the following abreviatures:

S = singlet
d = doublet
t = triplet
q = quadruplet
sc = complex signal
sa = broad signal

The number of protons corresponding to each signal, obtained by electronic integration, is indicated between brackets.

In addition, in said TABLE 1 some columns are headed by alphabetical signs, with the following significance:

Column A = No. of the example

Column B = Brief denomination of the compound obtained and analyzed.

Column C = Indicates the No. corresponding to the former example (1-4) in which the method of obtention has been described in detail.

Column D = Melting point in ºC

(*) = as maleate
(**) = as hydrochloride

TABLE 1

| A | B | R | C | D | NMR |
|---|---|---|---|---|---|
| 3 | WAS 4207 | 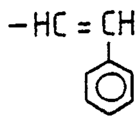 | 1 | 120-122 | 7,2/sc(13); 5,5-6,1/sc(3); 2,5-3,7/sc(16) |
| 4 | WAS-4220 | 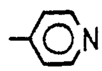 | 2 | 144(*) | 8,55/d(2); 7,45/d(2); 7,1/sc(8); 6,0/t(1); 2,5-4,0/sc(16) |
| 5 | WAS-4221 | 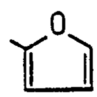 | 2 | 189(*) | 7,6/sc(1); 7,2/sc(10); 6,0/t(1); 2,5-4,0/sc(16) |
| 6 | WAS-4222 | 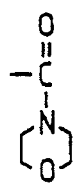 | 2 | 192(*) | 7,2/sc(89; 5,95/5(1); 2,5-3,75/ sc(22) |
| 7 | WAS-4223 | 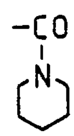 | 2 | 178(*) | 7,25/sc(8); 6,0/t(1); 1,5-4,0/sc(26) |
| 8 | WAS-4224 | 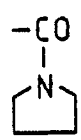 | 2 | 186(*) | 7,15/sc(8); 5,9/t(1); 1,6-3,7/sc(24) |

TABLE 1

| A | B | R | C | D | NMR |
|---|---|---|---|---|---|
| 9 | WAS-4237 | 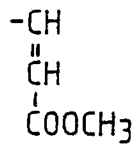 | 2 | 178(*) | 7,25/sc(8); 5,5-6,1/sc(3); 2,5-4/sc(19) |
| 10 | WAS-4238 | 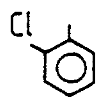 | 2 | 183(*) | 7,2/sc(12); 6,0/t(1); 2,5-4/sc(16) |
| 11 | WAS-4239 | 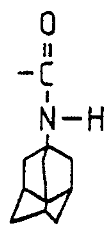 | 2 | 160(*) | 7,2/sc(8); 5,9/t(1); 4,9/sa(1); 2,5-4/sc(16); 1,6/sc(15) |
| 12 | WAS-4601 | 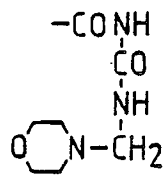 | 2 | 105(*) | 8,3/sa(2); 7,2/sc(8); 6,0/t(1); 2,5-4/sc(26) |
| 13 | WAS-4602 | 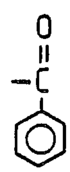 | 2 | 176(*) | 7,2/sc(13); 6,0/t(1); 2,5-4/sc(16) |
| 14 | WAS-4604 | 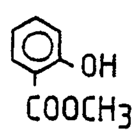 | 2 | 186(*) | 9,5/sa(1); 7,5/sc(1); 7,2/sc(10); 6,0/t(1); 2,5-4/sc(19) |
| 15 | WAS-4605 | 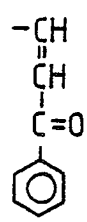 | 2 | 172(*) | 7,2/sc(13); 5,4-6,1/sc(3); 2,5-4/sc(16) |

TABLE 1

| A | B | R | C | D | NMR |
|---|---|---|---|---|---|
| 16 | WAS-4225 | 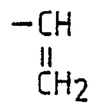 | 2 | 179(*) | 7,25/sc(8);<br>5,3-6,1/sc(4);<br>2,3-3,7/sc(16); |
| 17 | WAS-4227 | 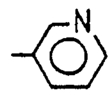 | 2 | 161(*) | 8,6/sc(2);<br>7,8/sc(2);<br>7,25/sc(8);<br>6,0/t(1);<br>2,5-3,7/sc(16) |
| 18 | WAS-4229 | $-CH_2-OH$ | 2 | 159(*) | 8,5/sa(1);<br>7,15/sc(8);<br>6,0/t(1);<br>2,5-4/sc(18) |
| 19 | WAS-4231 | 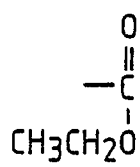 | 2 | 181(*) | 7,25/sc(8);<br>6,0/t(1);<br>1,8-4/sc(21) |
| 20 | WAS-4232 | 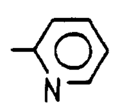 | 2 | 148(*) | 7,7-8,5/sc(4);<br>7,2/sc(8);<br>6,0/t(1);<br>2,5-4/sc(16) |
| 21 | WAS-4233 | $-COOH$ | 2 | 197(*) | 7,15/sc(8);<br>6,0/t(1);<br>2,5-3,5/sc(19) |
| 22 | WAS-4234 | 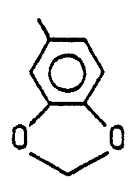 | 2 | 191(*) | 7,6/sc(1);<br>7,2/sc(10);<br>6,0/t(1);<br>2,5-4/sc(16) |

## PHARMACOLOGICAL PROPERTIES

The compounds object of the present invention possess vasodilatory activity. In the following, the methods used for evaluation of the above-mentioned pharmacological activity are described, together with the results obtained in each which correspond to the most representative compounds among those which are object of the present invention.

### Vasodilatory activity

The results corresponding to this trial are shown in TABLE 2 and TABLE 3.

The results have been symbolicaly expressed by crosses, the number of which is proportional to the intensity of effect, the maximum value of four crossess representing an intensity of action comparable to the standard drug used. The significance of the symbols is as follows:

+ + + + Very intense activity
+ + + Considerable activity
+ + Moderate acitivity
+ Low activity
0 Null activity
-- not tested

In order to test the vasodilatory activity, in the first trial the method employed was that of the isolated rat hindquarters (TABLE 2), which consists in antagonizing the vasoconstrictive effect of perfusion of hyperkalaemic Tyrode solution, according to the method of F.N. Fastier and F.H. Smirk (J. Pharm. Exp. Terap. 89, 256-270 (1947)), and calculating in this case, for the products which showed greatest activity, the $ED_{30}$, that is to say, the dose which produced a 30% vasodilatory activity with respect to the basic vasoconstriction.

As has already been said, in the rest of the less active products the activity is expressed in the form of crosses with respect to the standard drugs, in this case cinnarizine and flunarizine.

In a further trial, the vasodilatory activity has been evaluated by the method of vasodilatation in perfused rabbit cerebral territory described by P. Vaupel and H. Hutten (Arzneim. Forsch./Drug Res. 30(I), 598-602 (1980)), calculating the variation in perfusion pressure after treatment (TABLE 3).

In TABLE 3, the same conventional signs based on crosses, with the same relative significance, has been applied as in TABLE 2.

The standard drug employed was cinnarizine.

TABLE 2+3

| COMPOUND | COMPOUND DESCRIBED IN EXAMPLE N° | VASODILATORY ACTIVITY | | | |
|---|---|---|---|---|---|
| | | (Rat hind-quarters) | | (Rabbit cerebral territory | |
| | | EVALUATION | $ED_{30}$ x$10^{-5}$ M | EVALUATION | Vasodilatory Index [1] ($mm^2$) |
| WAS-4206 | 1 | + + + + | 1.93 | + + | 285.3 |
| WAS-4207 | 3 | + + + + | 2.17 | 0 | 16.2 |
| WAS-4220 | 4 | -- | -- | + + + + | 1159.2 |
| WAS-4221 | 5 | -- | -- | + + + | 321.4 |
| WAS-4222 | 6 | + | -- | + + + + | 454.6 |
| WAS-4223 | 7 | -- | -- | + + + + | 1133.6 |
| WAS-4224 | 8 | -- | -- | + + + | 342.0 |
| WAS-4225 | 16 | + + + + | 1.46 | -- (+ +) | -- (274.6) |
| WAS-4226 | 2 | 0 | -- | 0 | 74.0 |
| WAS-4237 | 9 | + + + + | 4.23 | 0 | 19.6 |
| WAS-4601 | 12 | + + + + | 4.33 | + | 180.8 |
| WAS-4602 | 13 | 0 | -- | + + + | 378.3 |
| WAS-4605 | 15 | + + + + | 2.97 | + + + + | 420.9 |
| Results obtained with the standard drugs | | | | | |
| Cinnarizine | | + + + + | 1.51 | + + + + | 535.0 |
| Flunarizine | | + + + + | 3.47 | -- | -- |

[1] Calculated by multiplying the % vasodilation by duration of action.

TOXICITY

In TABLE 4 the results of the indicative $LD_{50}$ for the most representative of the compounds described in the present invention are expressed.

The said compounds are administered by the intraperitoneal route to Swiss mice, observing the toxic effects, the calculating the mortality rate and the $LD_{50}$ 7 days post-administration.

TABLE 4

| COMPOUND | COMPOUND DESCRIBED IN EXAMPLE No. | INDICATIVE $LD_{50}$ mg/kg i.p. |
|---|---|---|
| WAS-4206 | ~~2~~ 1 [illegible] | 880 |
| WAS-4207 | ~~10~~ 3 | 925 |
| WAS-4220 | ~~21~~ 4 | 185 |
| WAS-4221 | ~~22~~ 5 | 185 |
| WAS-4222 | ~~23~~ 6 | 185 |
| WAS-4223 | ~~24~~ 7 | 185 |
| WAS-4224 | ~~25~~ 8 | 385 |

(Continued on the next page)

TABLE 4 (Cont.)

| COMPOUND | COMPOUND DESCRIBED IN EXAMPLE No. | INDICATIVE $LD_{50}$ - mg/kg i.p. |
|---|---|---|
| WAS-4225 | ~~26~~ 9 | 175 |
| WAS-4226 | 2 and 4 | 385 |
| WAS-4227 | ~~27~~ 10 | 175 |
| ~~WAS-4228~~ | ~~28~~ | ~~118~~ |
| WAS-4229 | ~~29~~ 11 | 115 |
| ~~WAS-4230~~ | ~~30~~ | ~~115~~ |
| WAS-4231 | ~~31~~ 12 | 175 |
| WAS-4232 | ~~32~~ 13 | 115 |
| WAS-4233 | ~~33~~ 14 | 900 |
| WAS-4234 | ~~34~~ 15 | 600 |
| ~~WAS-4235~~ | ~~35~~ | ~~3000~~ |
| ~~WAS-4236~~ | ~~36~~ | ~~385~~ |
| WAS-4237 | ~~37~~ 16 | 3000 |
| WAS-4238 | ~~38~~ 17 | 340 |
| WAS-4239 | ~~39~~ 18 | 340 |

(Continued on the next page)

TABLE 4 (Cont.)

| COMPOUND | COMPOUND DESCRIBED IN EXAMPLE No. | INDICATIVE $LD_{50}$ mg/kg i.p. |
|---|---|---|
| WAS-4601 | 19 | 125 |
| WAS-4602 | 20 | 200 |
| WAS-4604 | 21 | 900 |
| WAS-4605 | 22 | 115 |

THERAPEUTICAL APPLICATIONS

The compounds described in the present invention have the following therapeutical applications:

Treatment and prophylaxis of cerebral circulatory insufficiency. Cerebral vasulat accidents, hemiplegic sequaelae, prevention of relapse. Dizzyspells and Meunier-type syndromes. Protection of the brain against endogenous, toxic, endocrine, infections and drug-induced aggressions.

Complementary treatment of arterial hypertension. Prevention of capillary fragility and hyperpermeability. Basic treatment of geriatric cerebral vascular pathology. Peripheral vascular disorders: generalized arteriosclerosis and its symptoms. Ophthalmic and vestibular vascular disorders.

PHARMACEUTICAL FORMS AND DOSAGE

All the compounds object of the present invention can be administered under all the pharmaceutical forms compatible with their pharmacotechnical and therapeutic properties, at an adequate dosage.

## Claims

1. Derivatives of dihydrodibenzocycloheptiliden-ethyl-piperazine of the general formula

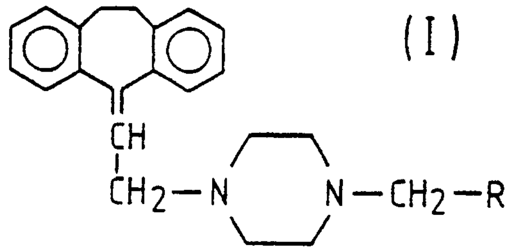

(I)

in which R represents

$-CH=CH_2$ ; $-CH_2OH$ ; $-CO_2H$ ; $-CO_2C_2H_5$ ; $-CH=CH-CO_2CH_3$ ;

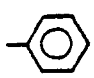 ; 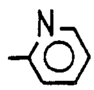 ; 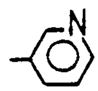 ; 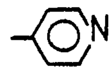 ; 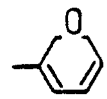 ; 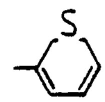 ;

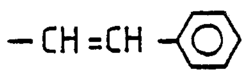 ; 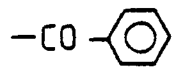 ; 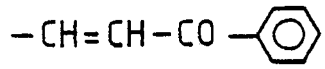 ;

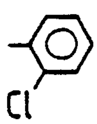 ; 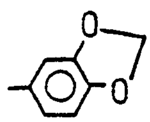 ; 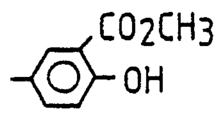 ; 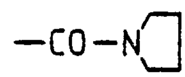 ;

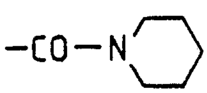 ; 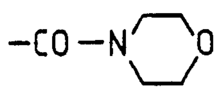 ; 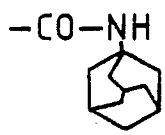 ; 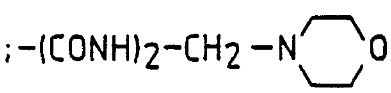

2. A process for preparing the compounds of general formula(I) of claim 1 by reaction of a piperazine of general formula

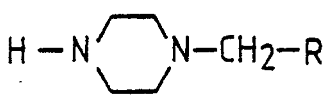

(II)

wherein R is defined as in claim 1, with a halogen derivative of the formula

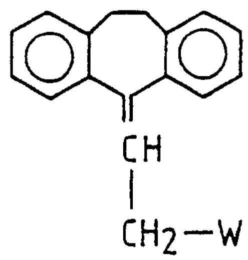

(III)

W: Cl, Br

wherein W is chlorine or bromine.

3. A pharmaceutical composition comprising as active ingredient a compound of the general formula (I) as defined as claim 1 together with a pharmaceutically acceptable carrier therefor.

4. A compound of the general formula (I) as defined in claim 1 for use as vasodilatory agent.

5. A derivative according to claim 1 wherein R is selected from ethylene, phenyl and 2-thienyl.